# EUROPEAN PATENT SPECIFICATION

(11) **EP 1 616 598 B1**
(45) Date of publication and mention of the grant of the patent: **11.03.2009**
(21) Application number: 04077066.1
(22) Date of filing: 16.07.2004
(51) Int. Cl.: A61N 5/10

(54) **Device for radiation treatment of proliferative tissue surrounding a cavity in an animal body**
Einrichtung zur Strahlenbehandlung von proliferativem Gewebe grenzend an eine Höhle in einem tierischen Körper.
Dispositif d'irradiation de tissu proliférant entourant une cavité d'un corps animal

(43) Date of publication of application: 18.01.2006
(73) Proprietor: Nucletron B.V., 3905 TH Veenendaal (NL)
(72) Inventor: Kindlein, Johann, 47918 Toenisvorst (DE); van Krieken, Frits, 6953 CL Dieren (NL)
(74) Representative: Valkonet, Rutger

(56) References cited:
- EP-A- 1 402 922
- US-A- 3 872 856
- US-A- 6 083 148
- US-A1- 2003 153 803

## Description

The invention relates to a device for radiation treatment of proliferative tissue surrounding a cavity in an animal body according to the preamble of claim 1.

Such device is for example known from US Patent No. 3,872,856 to Ralph S. Clayton and from European patent application no. 1 402 922 A1 in the name of the present applicant, Nucletron B.V.

The one major advance that has had the greatest influence on the re-emergence of brachytherapy in recent years has been the introduction of remote afterloaders. A remote afterloader (or afterloading apparatus) enables the insertion of energy emitting sources through a catheter tube towards a specific location within a patient's body without the risk of exposing unnecessary radiation doses to the radiotherapy staff.

In addition to this development there has been a trend in the past few years towards the use of high dose rate (HDR) energy emitting sources in brachytherapy applications in which much higher activity sources are inserted or implanted within a patient's body for much shorter periods of treatment time.

These HDR sources are sometimes inserted in a single fraction or more often with a few separate insertions.

Together with the increased interest for this treatment modality more sophisticated applicators have been developed and the classical plastic or metal applicators are replaced step by step with combined applicator-balloon or balloon-based devices. These devices are surrounded by an inflatable balloon system, which balloon system is introduced with the applicator into a natural body cavity or where a cancer tumour has been excised by means of surgery.

Following surgical removal of a tumour, the inflatable balloon system is introduced into the cavity caused by the removal of the tumour. After inflating the balloon system one or more energy emitting sources are introduced at one or more locations within the body cavity in order to treat the tissue surrounding said surgically excised tumour with radiation in order to kill any cancer cells that may be present in the margins surrounding the excised tumour.

An inflatable balloon system allows an immobilization of the region within the cavity the patient's body to be treated by radiation, or a centering of the treatment region, or it provides a displacement of organs at risk away from the treatment region to be irradiated.

From the above it will be clear that a correct positioning of the applicator and the reproducibility of the position of the applicator is important and will have a direct influence on the clinical outcome. The dose rate emitted by the energy emitting source inserted within the body cavity and present at a certain point will be determined from the distance of the source to the tissue and this distance is dependent from the inflation or deflation status of the balloon system applicator.

As the balloon system applicator is inserted inside a body cavity a visual control of the inflation status is impossible. The use of additional imaging techniques, like ultrasound or X-ray imaging, constitutes an extra discomforting burden for the patient and cannot be maintained during the radiation treatment when radiation is being delivered to the treatment region.

As result, with the inflatable balloon system devices presently known the risk of any misadministration of a radiation dose to the patient is very high and can not be controlled or corrected.

It is therefor an object of the invention to provide a device for radiation treatment of proliferative tissue surrounding a cavity in an animal patient body according to the above preamble capable in monitoring the real, actual functional status of the inflated balloon system present inside said body cavity, especially when said device is utilized with an after loading apparatus.

The device is according to the invention characterized according to claims 1.

Hence with the device according to the invention the actual inflation status of the inflated balloon system can be determined, providing accurate information about the operational conditions of the device during radiation treatments being performed in an animal body. Any malfunction can be easily detected thereby obviating the risk of any misadministration of a radiation dose to the patient.

More in particular these features allow any correction of any unfavourable operational conditions, which may adversely affect the radiation treatment being performed in the cavity of said animal body.

Especially in one preferred embodiment said radiation delivering means are arranged in retracting said at least one energy emitting source from said cavity based on control signals generated by said monitoring means as a result of said inflation status comparison. This safe measure prevents an uncontrolled radiation dose being administered to the animal body (patient) during a malfunction of the balloon system.

In another preferred embodiment said at least one energy emitting source is an activatable energy emitting source and wherein said radiation delivering means are arranged in de-activating said at least one energy emitting source in said cavity based on control signals generated by said monitoring means as a result of said inflation status comparison. Therefor the device according to the invention is not only suitable for energy emitting sources who emit radioactive radiation according to the principle of natural radioactive decay, but also for energy emitting sources who are to be activated in order to emit radiation, like an X-ray emitter, or laser source, etc.

In another preferred embodiment said inflation means are arranged in inflating and/or deflating the balloon system based on control signals generated by said monitoring means as a result of said inflation status comparison. This feature allows the inflation status of the balloon system to be corrected within a safety pressure bandwidth.

In this latter embodiment said monitoring means may comprise at least one pressure sensor for generating pressure data corresponding to the actual pressure of said pressurized medium in said inflated balloon system, said pressure data being used for said inflation status comparison.

More in particular the monitoring means are arranged in comparing said pressure data with a pre-determined pressure bandwidth and in operating the device based on control signals generated as a result of said pressure comparison.

In preferred embodiments said at least one pressure sensor can be positioned inside or outside the balloon system,

In another advantageous embodiment of the device according to the invention said monitoring means comprise an imaging device for generating image data corresponding to the actual balloon wall contour of the inflated balloon system, said image data being used for said inflation status comparison.

The monitoring means are thereby arranged in comparing said image data with a pre-determined balloon wall contour and in operating the device based on control signals generated as a result of said contour comparison.

Hence with the use of image data instead of pressure data the actual inflation contour of the balloon system inside the cavity in the patient's body is monitored and used for correcting the device in the event that any malfunction or deviation from the optimal operational conditions are detected.

In order to obtain more accurate information concerning the actual operational conditions of the device and the balloon system in a specific embodiment the monitoring means are arranged in converting said image data obtained with said imaging device into a three-dimensional image of the actual balloon wall contour of the inflated balloon system.

Said imaging device may be constructed as ultrasound imaging probe or as a video camera, which imaging devices are in a preferred embodiment insertable inside the balloon system in order to obtain more actual local information allowing a more accurate and prompt correction of the operational status of the device in case of a malfunction or deviation.

In yet another advantageous embodiment said monitoring means comprises at least one radiation dose sensor for generating radiation data corresponding to measured radiation emitted by said at least one energy emitting source being placed within said cavity and corresponding to the actual distance between said at least one radiation dose sensor and said at least one energy emitting source within said cavity, said radiation data being used for said inflation status comparison.

More in particular the monitoring means are arranged in comparing said radiation data with a pre-determined desired distance between said at least one radiation dose sensor and said at least one energy emitting source within said cavity and in operating the device based on control signals generated as a result of said radiation comparison.

The radiation data being generated by the radiation sensor is a measure of the actual distance between the sensor connected to the balloon system and the energy emitting source placed within the cavity and which source emits the radiation that is detected with the radiation sensor. The radiation intensity of emitted radiation decreases with the distance according to pre-determined rules. By comparing the actual distance as obtained from the measured radiation with a pre-determined desired distance accurate information can be obtained about the actual inflation status of the balloon system.

In the event the balloon system becomes deflated due to a malfunction the radiation being detected by the sensor will reveal a shorter or decreased distance between the sensor and the source emitting said radiation. Also in the event of an over-inflating of the balloon system the radiation being detected by the sensor will reveal a longer or increased distance. In both situations the device is controlled in order to correct for these malfunctions or deviations from the optimal operational conditions.

Preferably said at least one radiation sensor is connected to the inner or outer wall of the balloon system.

Furthermore the inflation means may comprise a piston-cylinder combination having a cylinder and a piston movable accommodated in said cylinder. More in particular said inflation means comprise piston drive means for displacing said piston within said cylinder based on control signals generated by said monitoring means.

Furthermore a medium conduct is present interconnecting the cylinder with the inflatable balloon, whereby an additional feature consists of a supply vessel for said medium being present in said medium conduct.

More in particular a first valve is accommodated in said medium conduct between said supply vessel and said piston-cylinder combination, whereas a second valve is accommodated in said medium conduct between said piston-cylinder combination and said inflatable balloon system. As in a specific embodiment both said first and said second valve can be actuated by said monitoring means the inflation status of the balloon system can be accurately controlled and any malfunction can be detected and direct specific measures can be performed avoiding the misadministration of a radiation dose to the patient.

In another specific embodiment said radiation delivering means are constructed as an after loading apparatus.

The invention will now be described with reference to a drawing, which shows in:
Figure 1 a first embodiment of a device according to the invention;
Figure 2 a second embodiment of a device according to the invention;
Figure 3 a third embodiment of a device according to the invention;
Figures 4a-4b a fourth embodiment of a device according to the invention.

For the sake of clarity corresponding parts of the embodiments shown in the enclosed drawings are depicted with identical reference numerals.

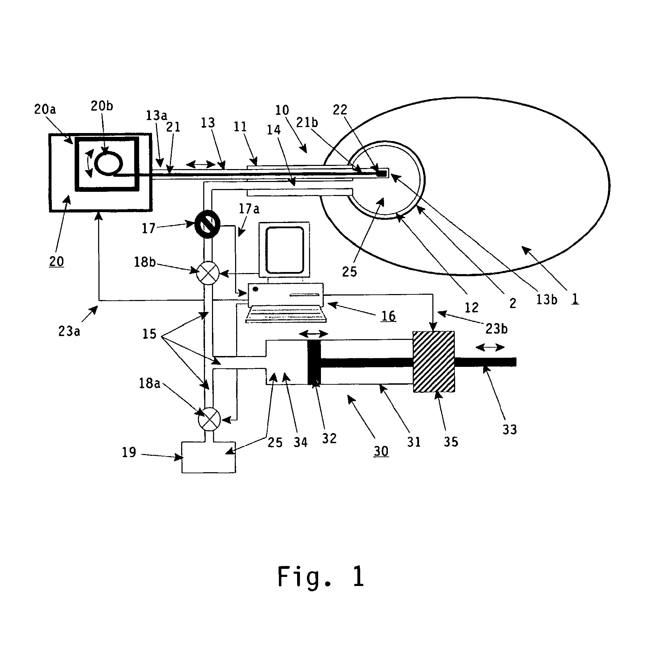

Figure 1 discloses a lateral view of an embodiment of a device for radiation treatment of proliferative tissue surrounding a cavity in an animal body according to the invention. In this drawing a part of the animal body is depicted with reference numeral 1, for example the head of a patient, or a breast of a woman. A cancer tumour has been removed from said part 1 of said animal body during a surgical procedure a cavity 2. As any cancer cell may still be present in the margins surrounding the surgically excised tumour in said cavity 2 a radiation treatment of said cancer cell is desirable with the use of radioactive emissions from energy emitting sources positioned inside said cavity 2.

To this end an applicator 10 is introduced into the cavity 2, which device 10 comprises a supportive probe 11 having an inflatable balloon system 12 connected to a distal end 11a of said supportive probe 11. Once the deflated balloon system 12 has been introduced inside the cavity 2, the balloon system 12 is inflated by suitable inflation means by injecting a pressurized medium 25 (for example a fluid) via a passageway 14 in the supportive probe 11 towards the distensible reservoir formed by the balloon system 12.

Said pressurized medium 25 could be a fluid or a gaseous medium or a liquid containing radioactive particles. Also other type of pressurized media, radioactive or not, can be utilized.

In addition, the supportive probe 11 is provided with a guidance channel through which a flexible catheter tube 13 is guidable until it extends with its distal end 13b within the cavity 2. The catheter tube 13 is connected with its proximal end 13a with radiation delivery means 20, here a remote afterloader apparatus 20 for performing radiation therapy treatments of the cancer tissue surrounding the cavity 2. The afterloader apparatus 20 contains a radiation shielded compartment 20a, in which compartment an energy emitting source 22 is accommodated.

The energy emitting source 22 is attached to a distal end 21b of a source wire 21, which source wire can be advanced through the hollow catheter tube 13 by means of wire drive means 20b. The energy emitting source 22 can be advanced from said radiation shielded compartment 20a through the hollow catheter tube 13 towards a desired location within the cavity 2.

The positioning of the energy emitting source 22 at different locations within its hollow catheter tube 13 and in the cavity 2 gives more possibilities for performing a radiation therapy treatment session. The total dose distribution of the tissue to be treated will be conformal with the volume of the tumour tissue surrounding the cavity 2 by optimizing the dwell times for the different positions within the cavity 2 of the energy emitting source 22. Moreover the guidance of the energy emitting source 22 through the hollow catheter tube 13 within the cavity 2 allows a temporarily insertion of the source 22 in the reproducible manner at different locations.

With the use of an afterloader device 20 it is possible to use the device 10 according to the invention to perform radiation therapy treatment sessions with so called High Dose Rate (HDR) or Pulse Dose Rate (PDR) in emitting sources, which requires special and save handling prior to each treatment session. These HDR or PDR sources are characterised by a high radiation intensity profile and are thus for safety reasons accommodated in a radiation shielded compartment 20a within the afterloader 12. A radiation therapy treatment session with such high intensity energy emitting sources requires specific proceedings concerning handling a storage of these sources.

To this end in a proper operation of the inflatable balloon system 12 is necessary in order to avoid any misadministration of a radiation dose to the patient. The device 10 according to the invention comprises monitory means for monitoring the inflation status of the inflatable balloon system 12. More particular said monitoring means 16 uses at least one pressure sensor 17 which is accommodated in a medium conduct 15. The pressure sensor 17 senses the actual pressure of the pressurized medium 25 inside the inflate balloon system 12. Said sensor 17 generates a pressure signal 17a conformal with the pressure being sensed and said pressure signal 17a is fed to the monitoring means 16.

According to the invention said monitoring means 16 are arranged in comparing said pressure being sensed by the pressure sensor 17 with a predetermined pressure bandwidth. Said predetermined pressure bandwidth describes a range of pressures of said pressurized medium 25 under which pressure circumstances the device 10 according to the invention can be operated under normal conditions.

In the event a significant deviation of the pressure being sensed by said pressure sensor 17 and the predetermined pressure bandwidth is detected the monitoring means 12 are arranged in controlling the device 10 based on control signals generated as a result of said pressure comparison.

In other words, in the event the pressure being sensed by the pressure sensor 17 lies outside the predetermined pressure bandwidth the monitoring means 16 will generate suitable control signals based on which the device 10 will be controlled. In a first control step a control signal 23a will be generated by the monitoring means 16 and fed to the afterloader apparatus 20 in order to actuate the wire drive means 20b. For example in the event that the pressure of the pressurized medium 25 inside the balloon system 12 becomes significantly low (for example due to a leakage) the control signal 23a generated by the monitoring means 16 will result in an immediate retraction of the source wire 21 and the emitting source 22 by the wire drive means 20b.

In another embodiment (not depicted) the energy emitting source 22 is an activatable source, like an X-ray emitting source or laser device and in the event of a malfunction the device according to the invention (and in particular the afterloader 20) is arranged in de-activating the energy emitting source within the cavity 2.

These safe measures prevent an uncontrolled radiation dose being administered to the cancer tumour surrounding the cavity 2 inside the animal body 1 during a malfunction (leakage) of the balloon system 2.

In an other control step a control signal 23b generated by the monitoring means 16 will be fed to the inflation means 30 in order to actuate the inflation means such that the balloon system 12 is inflated or deflated until the medium pressure of the medium 25 present in the balloon system 12 will fall within the predetermined pressure bandwidth corresponding with the optimal operation conditions.

A specific embodiment of the inflation means is here described by way of example. However it should be note that also other types of inflation means are suitable and can be implemented in the device according to the invention. The inflation means 30 in this example comprise a piston-cylinder combination 30 having a cylinder 31 and a piston 32 which is movable accommodated in said cylinder 31. The piston-cylinder combination 30 is provided with piston drive means 35 for displacing said piston 32 within the cylinder 31. To this end the piston 32 is mounted on a piston rod 33. The displacement of the piston 32 inside the cylinder 31 by the piston drive means 35 takes place based on control signals generated by the monitoring means 16.

The piston-cylinder combination 30 is provided with an cylinder chamber 34 in which the amount of medium 25 for inflating the balloon system 12 of the device 10 according to the invention is accommodated. The piston-cylinder combination 30 is connected with the passage way 14 and the inflatable balloon system 12 by means of a medium conduct 15. In said medium conduct 15 a supply vessel 19 is accommodated which is suited for storing a certain amount of medium 25. The storage vessel 19 is closed by means of a first valve 18a which is accommodated in the medium conduct 15 between the piston-cylinder combination 30 and the storage vessel 19. Between the piston-cylinder combination 30 and the passage way 14/the balloon system 12 a second valve 18b is accommodated in the medium conduct 15.

Both valves 18a and 18b can be actuated by the monitoring means 16 with the use of suitable control signals. For example in the event that the balloon system 12 needs to be inflated (for example as a result of a possible decrease in the operational pressure inside the balloon system 12) the monitoring means 16 generate suitable control signals based on the comparison between the actual sensed low pressure inside the balloon system 12 and the predetermined optimal pressure bandwidth. These control signals will effect a closure of the first valve 18a and an opening of the second valve 18b. A likewise actuation of the piston drive means 35 will be effected, such that medium 25 present in the cylinder chamber 34 is pushed through the conduct 15 through the open second valve 18b and the passage way 14 towards the balloon system 12, thereby inflating the balloon system 12.

Likewise, in the event that a too high pressure inside the balloon system is sensed suitable control signals generated by the monitoring means 16 will actuate the piston drive means 35 such that the piston 32 is retracted inside the cylinder 31 thereby redrawing or deflating medium 25 out of the balloon system 12 and the passage way 14 towards the cylinder chamber 34. With this control step the actual pressure inside the balloon system 12 will decrease.

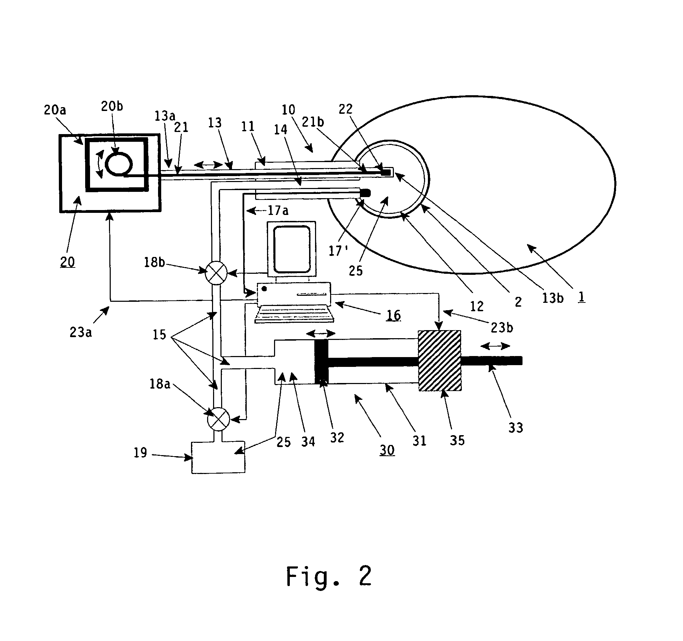

In Figure 2 a further embodiment of a device according to the invention is disclosed wherein a pressure sensor 17' is accommodated inside the balloon system 12 for detecting the actual pressure of the pressurized medium 25 within the balloon system 12. The pressure sensor 17' generates pressure data which are fed via a signal line 17a to the monitoring means 16. The monitoring means 16 operate in the similar way as described in relation to the embodiment of Figure 1.

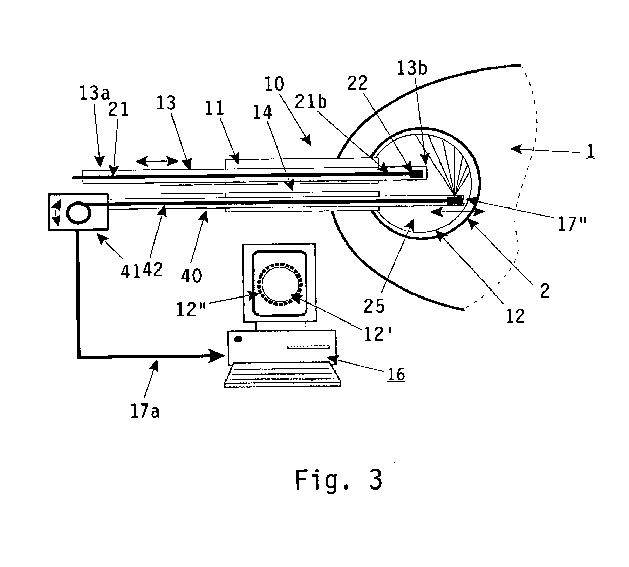

In Figure 3 another embodiment of a device according to the invention is described, wherein an imaging device 17" is used for generating image data, which correspond to the actual contour or shape of the balloon wall of the inflated balloon system 12. The image data generated by the imaging device 17" is fed via a signal line 42 and 17a towards the monitoring means 16, which operate in a similar way as described in relation to the embodiments of Figures 1 and 2.

The imaging device 17" in the embodiment as disclosed in Figure 3 is placed inside the balloon system 12 via an insertion catheter 40, which is guided through an appropriate insertion channel (not depicted) present in the supportive probe 11 until within the balloon system 12. The imaging device 17" is connected to a signal cable 42, which is inserted and retracted through the insertion catheter 40 until within the balloon system 12 using suitable drive means 41.

In a first specific embodiment of the device as disclosed in Figure 3 the imaging device 17" is inserted with the use of the signal cable 42 and the drive means 41 towards a specific position inside the balloon system 12 for example a centre position in the middle of the balloon system (not depicted). Subsequently the imaging device 17" generates image data in one measurement "sweep" covering the whole actual balloon wall contour of the balloon system 12.

In other functional embodiment the imaging device 17" is advanced using the signal cable 42 by the drive means 41 in a step wise manner through the insertion catheter 40 through the balloon system 12 thereby generating image data in sequential measurements or "sweeps" of the actual balloon wall contour of the balloon system 12.

The image data representing the actual balloon wall contour of the balloon system 12 is fed via the signal cable 42 and 17a towards the monitoring means 16, wherein said image data is converted into a three dimensional balloon wall contour indicated with reference numeral 12'.

According to the method of the invention the monitoring means 16 are arranged in comparing said three dimensional balloon wall contour 12' with a pre-determined balloon wall contour as depicted as a dashed circle and reference numeral 12". Based on this contour comparison using the image data obtained with the imaging device 17" any deviations or malfunctions of the balloon wall 12 are quickly detected and appropriate control can be performed in order to correct for the malfunctions being detected.

Likewise the imaging device 17" can be positioned outside the cavity 2 and the patient's body 1 in order to generate an image of the cavity 2 using suitable imaging techniques.

The imaging device 17" (placed inside or outside the cavity 2 in the patient's body 1) can be an ultrasound imaging probe or a video camera. Especially an ultrasound imaging probe or a video camera can be constructed in small dimensions in order to allow an insertion through the insertion catheter 40 until within the inflated balloon system 12.

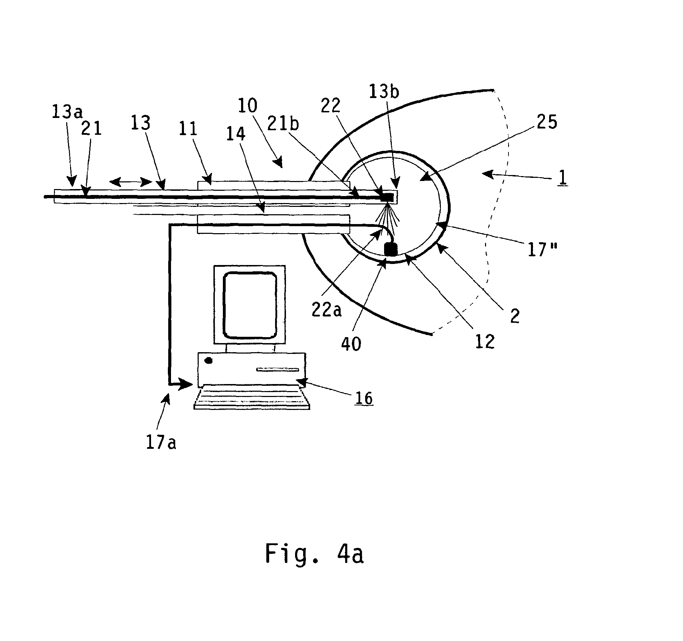

In Figures 4a-4b another embodiment of a device according to the invention is described, wherein a radiation sensor 40 is used for generating radiation data based on detected radiation 22a as emitted by the energy emitting source 22. Based on the general principle that the radiation intensity being emitted decreases with the distance, the radiation 22a being detected corresponds to the actual distance between the sensor 40 and the energy emitting source 22.

As the radiation sensor is connected to the inner or outer wall of the balloon system 12 the distance between the sensor 40 and the energy emitting source 22 being placed within the cavity 2 is dependent from the inflation status of the balloon system.

In Figure 4a the ideal operational condition of the device according to the invention is depicted, where the balloon system 12 is inflated such that it is conformal with the inner dimensions of the cavity 2. The distance between the radiation sensor 40 and the source 22 is optimal for performing radiation treatments and the radiation being emitted by the source 22 and detected by the sensor 40 corresponds to the pre-determined optimal distance, when the balloon system is inflated as in Figure 4a.

The radiation data generated by the sensor 40 is fed via the signal line 17a towards the monitoring means 16, which operate in a similar way as described in relation to the embodiments of Figures 1, 2 and 3. In the situation of Figure 4a a comparison by the monitoring means 16 between the actual distance being detected and a pre-determined distance will reveal no malfunction concerning the inflation status of the device.

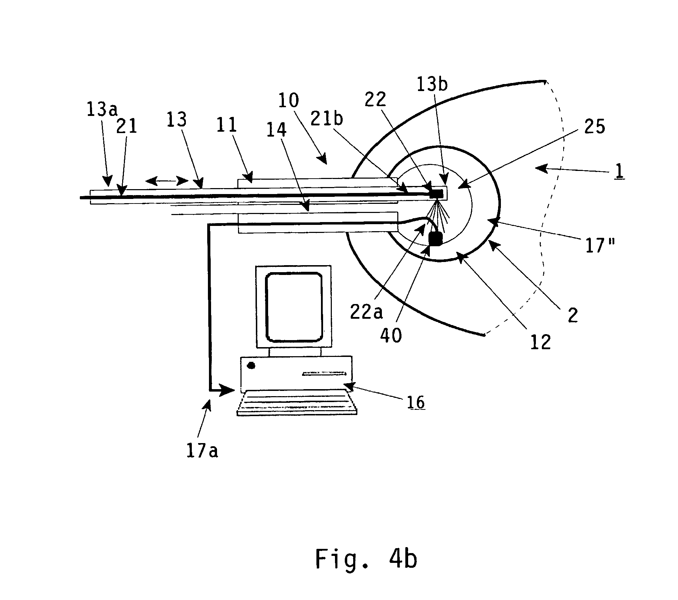

However, Figure 4b discloses a malfunction wherein the balloon system becomes deflated. The radiation 22a being detected by the sensor 40 will reveal a shorter or decreased distance between the radiation sensor 40 and the source 22 emitting said radiation 22a. Said distance deviation will be detected during the "real time" radiation comparison as performed by the monitoring means 16 and suitable control signals 23a or 23b (see Figures 1 and 2) will be generated and fed to the source wire drive means 20a of the afterloader 20 or to the inflation means 30 in order to correct for this malfunction.

Also in the event of an over-inflating of the balloon system 12 the radiation 22a being detected by the sensor 40 will reveal a longer or increased distance and also a suitable control of the device is performed by the monitoring means 16 in order to correct for this malfunction.

It will be appreciated that with the device according to the invention a more safe operation of a device according to the invention with the use of an afterloader apparatus is obtained wherein possible hazardous operational conditions are avoided as with a continuous monitoring of the actual pressure inside the balloon system 12 possible changes in the operational pressure will be sensed immediately and a suitable control of the device 10 according to the invention is performed by the monitoring means 16 in order to correct for the change in the operational status of the device (balloon system 12).

## Claims

1. Device (10) for radiation treatment of proliferative tissue surrounding a cavity (2) in an animal body (1) comprising:
- at least one inflatable balloon system (12) having a balloon wall for placement in said cavity;
- inflation means (30) for inflating and deflating said balloon system (12) with a pressurized medium (25);
- radiation delivering means (20) for placing at least one energy emitting source (22) within said cavity (2) for performing said radiation treatment, as well as monitoring means (16) for monitoring the inflation status of the inflatable balloon system (12),
**characterized in that,** said monitoring means (16) are arranged to compare the actual inflation status being monitored with a pre-determined desired inflation status of the inflatable balloon system (12) and to operate the device (10) based on control signals generated as a result of said inflation status comparison.

2. Device (10) according to claim 1, **characterized in that,** said radiation delivering means (20) are arranged to retract said at least one energy emitting source (22) from said cavity (2) based on control signals generated by said monitoring means (16) as a result of said inflation status comparison.

3. Device (10) according to claim 1, **characterized in that,** said at least one energy emitting source (22) is an activatable energy emitting source and wherein said radiation delivering means (20) are arranged to de-activate said at least one energy emitting source in said cavity (2) based on control signals generated by said monitoring means (16) as a result of said inflation status comparison.

4. Device (10) according to claim 1, **characterized in that,** said inflation means (30) are arranged to inflate and/or deflate the balloon system (12) based on control signals generated by said monitoring means (16) as a result of said inflation status comparison.

5. Device (10) according to any one of the claims 1-4, **characterized in that,** said monitoring means (16) comprises at least one pressure sensor (17-17') for generating pressure data corresponding to the actual pressure of said pressurized medium (25) in said inflated balloon system (12), said pressure data being used for said inflation status comparison.

6. Device (10) according to claim 5, **characterized in that,** the monitoring means (16) are arranged to compare said pressure data with a pre-determined pressure bandwidth and to operate the device (10) based on control signals generated as a result of said pressure comparison.

7. Device (10) according to claim 5 or 6, **characterized in that,** said at least one pressure sensor (17') is positioned inside the balloon system (12).

8. Device according to claim 5 or 6, **characterized in that,** said at least one pressure sensor (17) is positioned outside the balloon system.

9. Device (10) according to any one of the claims 1-4, **characterized in that,** said monitoring means (16) comprises an imaging device (17") for generating image data corresponding to the actual balloon wall contour of the inflated balloon system (12), said image data being used for said inflation status comparison.

10. Device (10) according to claim 9, **characterized in that,** the monitoring means (16) are arranged to compare said image data with a pre-determined balloon wall contour and to operate the device based on control signals generated as a result of said contour comparison.

11. Device (10) according to claim 9 or 10, **characterized in that,** the monitoring means (16) are arranged to convert said image data obtained with said imaging device (17") into a three-dimensional image of the actual balloon wall contour of the inflated balloon system (12).

12. Device (10) according to any one of the claims 9-11, **characterized in that,** said imaging device (17") is constructed as an ultrasound imaging probe.

13. Device (10) according to any one of the claims 9-11, **characterized in that,** said imaging device (17") is constructed as a video camera.

14. Device (10) according to any one of the claims 9-13, **characterized in that,** said imaging device (17") is insertable inside the balloon system (12).

15. Device (10) according to any one of the claims 1-4, **characterized in that,** said monitoring means (16) comprises at least one radiation dose sensor (40) for generating radiation data corresponding to measured radiation emitted by said at least one energy emitting source (22) being placed within said cavity (2) and corresponding to the actual distance between said at least one radiation dose sensor (40) and said at least one energy emitting source (22) within said cavity (2), said radiation data being used for said inflation status comparison.

16. Device (10) according to claim 15, **characterized in that,** the monitoring means (16) are arranged to compare said radiation data with a pre-determined desired distance between said at least one radiation dose sensor (40) and said at least one energy emitting source (22) within said cavity (2) and to operate the device (10) based on control signals generated as a result of said radiation comparison.

17. Device (10) according to claim 15 or 16, **characterized in that,** said at least one radiation sensor (40) is connected to the inner wall of the balloon system (12).

18. Device (10) according to claim 15 or 16, **characterized in that,** said at least one radiation sensor (40) is connected to the outer wall of the balloon system (12).

19. Device (10) according to any one of the preceding claims, **characterized in that,** the inflation means (30) comprise a piston-cylinder combination (31-32) having a cylinder (31) and a piston (32) movable accommodated in said cylinder.

20. Device (10) according to claim 19, **characterized in that,** said inflation means (30) comprise piston drive means (35) for displacing said piston (32) within said cylinder (31) based on control signals generated by said monitoring means (16).

21. Device (10) according to claim 20, **characterized in that,** a medium conduct 14-15) is present interconnecting the cylinder (31) with the inflatable balloon (12).

22. Device (10) according to claim 21, **characterized in that,** a supply vessel (19) for said medium (25) is present in said medium conduct (15).

23. Device (10) according to claim 22, **characterized in that,** a first valve (18a) is accommodated in said medium conduct (15) between said supply vessel (19) and said piston-cylinder combination (31-32).

24. Device (10) according to any one of the claims 21-23, **characterized in that,** a second valve (18b) is accommodated in said medium conduct (15) between said piston-cylinder combination (31-32) and said inflatable balloon system (12).

25. Device (10) according to claim 24, **characterized in that,** both said first (18a) and said second (18b) valve can be actuated by said monitoring means (16).

26. Device (10) according to any one of the preceding claims, **characterized in that,** said radiation delivering means (20) are constructed as an after loading apparatus.

27. Device (10) according to any one of the preceding claims, **characterized in that,** said pressurized medium (25) is a fluid, a gaseous medium or a liquid containing radioactive particles.

## Patentansprüche

1. Vorrichtung (10) zur Strahlenbehandlung von proliferativem Gewebe grenzend an einen Hohlraum (2) in einem tierischen Körper (1) mit:
wenigstens einem aufblasbaren Ballonsystem (12)mit einer Ballonwand zur Einbringung in den Hohlraum;
einer Aufblaseinrichtung (30) zum Aufblasen und Entleeren des Ballonsystems (12) mit einem druckbeaufschlagten Medium (25);
einer Strahlungsfreisetzungseinrichtung (20) zum Einbringen wenigstens einer Energie emittierenden Quelle (22) in den Hohlraum (2) zum Durchführen der Strahlenbehandlung, sowie
einer Beobachtungseinrichtung (16) zum Beobachten des Aufblasstatus des aufblasbaren Ballonsystems (12),
**dadurch gekennzeichnet, dass** die Beobachtungseinrichtung (16) angeordnet ist, um den beobachteten aktuellen Aufblasstatus mit einem vorbestimmten gewünschten Aufblasstatus des aufblasbaren Ballonsystems (12) zu vergleichen und um die Vorrichtung (10) basierend auf Steuersignalen zu betätigen, die als ein Ergebnis des Aufblasstatusvergleichs erzeugt werden.

2. Vorrichtung (10) gemäß Anspruch 1,
**dadurch gekennzeichnet, dass**
die Strahlungsfreisetzungseinrichtung (20) angeordnet ist, um die wenigstens eine Energie emittierende Quelle (22) aus dem Hohlraum (2) basierend auf Steuersignalen zurückzuziehen, die durch die Beobachtungseinrichtung (16) als ein Ergebnis des Aufblasstatusvergleichs erzeugt werden.

3. Vorrichtung gemäß Anspruch 1,
**dadurch gekennzeichnet, dass**
die wenigstens eine Energie emittierende Quelle (22) eine aktivierbare Energie emittierende Quelle ist, und wobei die Strahlungsfreisetzungseinrichtung (20) angeordnet ist, um die wenigstens eine Energie emittierende Quelle in dem Hohlraum (2) basierend auf Steuersignalen zu deaktivieren, die durch die Beobachtungseinrichtung (16) als ein Ergebnis des Aufblasstatusvergleichs erzeugt werden.

4. Vorrichtung (10) gemäß Anspruch 1,
**dadurch gekennzeichnet, dass**
die Aufblaseinrichtung (30) angeordnet ist, um das Ballonsystem (12) basierend auf Steuersignalen aufzublasen und/oder zu entleeren, die durch die Beobachtungseinrichtung (16) als ein Ergebnis des Aufblasstatusvergleichs erzeugt werden.

5. Vorrichtung (10) gemäß einem der Ansprüche 1 bis 4,
**dadurch gekennzeichnet, dass**
die Beobachtungseinrichtung (16) wenigstens einen Drucksensor (17 - 17') zum Erzeugen von Druckdaten entsprechend dem tatsächlichen Druck des druckbeaufschlagten Mediums (25) in dem aufgeblasenen Ballonsystem (12) aufweist, wobei die Druckdaten für den Aufblasstatusvergleich verwendet werden.

6. Vorrichtung (10) gemäß Anspruch 5,
**dadurch gekennzeichnet, dass**
die Beobachtungseinrichtung (16) angeordnet ist, um die Druckdaten mit einer vorbestimmten Druckbandbreite zu vergleichen und die Vorrichtung (10) basierend auf Steuersignalen zu betätigen, die als ein Ergebnis des Druckvergleichs erzeugt werden.

7. Vorrichtung (10) gemäß Anspruch 5 oder 6,
**dadurch gekennzeichnet, dass**
der wenigstens eine Drucksensor (17') im Inneren des Ballonsystems (12) positioniert ist.

8. Vorrichtung gemäß Anspruch 5 oder 6,
**dadurch gekennzeichnet, dass**
der wenigstens eine Drucksensor (17) außerhalb des Ballonsystems positioniert ist.

9. Vorrichtung (10) gemäß einem der Ansprüche 1 bis 4,
**dadurch gekennzeichnet, dass**
die Beobachtungseinrichtung (16) eine bildgebende Vorrichtung (17") zum Erzeugen von Bilddaten entsprechend der aktuellen Ballonwandkontur des aufgeblasenen Ballonsystems (12) aufweist, wobei die Bilddaten für den Aufblasstatusvergleich verwendet werden.

10. Vorrichtung (10) gemäß Anspruch 9,
**dadurch gekennzeichnet, dass**
die Beobachtungseinrichtung (16) angeordnet ist, um die Bilddaten mit einer vorbestimmten Ballonwandkontur zu vergleichen und die Vorrichtung basiert auf Steuersignalen zu betätigen, die als ein Ergebnis des Konturvergleichs erzeugt werden.

11. Vorrichtung (10) gemäß einem der Ansprüche 9 oder 10,
**dadurch gekennzeichnet, dass**
die Beobachtungseinrichtung (16) angeordnet ist, um die mit der bildgebenden Vorrichtung (17") erhaltenen Bilddaten in ein dreidimensionales Bild der tatsächlichen Ballonwandkontur des aufgeblasenen Ballonsystems (12) umzuwandeln.

12. Vorrichtung (10) gemäß einem der Ansprüche 9 bis 11,
**dadurch gekennzeichnet, dass**
die bildgebende Vorrichtung (17") als eine Ultraschall-Bildgebungssonde ausgebildet ist.

13. Vorrichtung (10) gemäß einem der Ansprüche 9 bis 11,
**dadurch gekennzeichnet, dass**
die bildgebende Vorrichtung (17") als eine Videokamera ausgebildet ist.

14. Vorrichtung (10) gemäß einem der Ansprüche 9 bis 13,
**dadurch gekennzeichnet, dass**
die bildgebende Vorrichtung (17") in das Innere des Ballonsystems (12) einführbar ist.

15. Vorrichtung (10) gemäß einem der Ansprüche 1 bis 4,
**dadurch gekennzeichnet, dass**
die Beobachtungseinrichtung (16) wenigstens einen Strahlungsdosissensor (40) zum Erzeugen von Strahlungsdaten entsprechend einer gemessenen Strahlung, die durch die wenigstens eine Energie emittierende Quelle (22) emittiert wird, welche im Inneren des Hohlraums (2) platziert ist, und entsprechend dem tatsächlichen Abstand zwischen dem wenigstens einen Strahlungsdosissensor (40) und der wenigstens einen Energie emittierenden Quelle (22) in dem Hohlraum (2) aufweist, wobei die Strahlungsdaten zum Aufblasstatusvergleich verwendet werden.

16. Vorrichtung (10) gemäß Anspruch 15,
**dadurch gekennzeichnet, dass**
die Beobachtungseinrichtung (16) angeordnet ist, um die Strahlungsdaten mit einem vorbestimmten gewünschten Abstand zwischen dem wenigstens einen Strahlungsdosissensor (40) und der wenigstens einen Energie emittierende Quelle (22) in dem Hohlraum (2) zu vergleichen und um die Vorrichtung (10) basiert auf Steuersignalen zu betätigen, die als ein Ergebnis des Strahlungsvergleichs erzeugt werden.

17. Vorrichtung (10) gemäß Anspruch 15 oder 16,
**dadurch gekennzeichnet, dass**
der wenigstens eine Strahlungssensor (40) mit der Innenwand des Ballonsystems (12) verbunden ist.

18. Vorrichtung (10) Anspruch 15 oder 16,
**dadurch gekennzeichnet, dass**
der wenigstens eine Strahlungssensor (40) mit der Außenwand des Ballonsystems (12) verbunden ist.

19. Vorrichtung (10) gemäß einem der vorangehenden Ansprüche,
**dadurch gekennzeichnet, dass**
die Aufblaseinrichtung (30) eine Kolben-Zylinder-Kombination (31- 32) aufweist, die einen Zylinder (31) und einen Kolben (32) hat, der bewegbar in dem Zylinder aufgenommen ist.

20. Vorrichtung (10) gemäß Anspruch 19,
**dadurch gekennzeichnet, dass**
die Aufblaseinrichtung (30) eine Kolbenantriebseinrichtung (35) zum Verschieben des Kolbens (32) in dem Zylinder (31) basierend auf Steuersignalen aufweist, die durch die Beobachtungseinrichtung (16) erzeugt werden.

21. Vorrichtung (10) gemäß Anspruch 20,
**dadurch gekennzeichnet, dass**
eine Medienleitung (14 - 15) vorhanden ist, welche den Zylinder (31) mit dem aufblasbaren Ballon (12) verbindet.

22. Vorrichtung (10) gemäß Anspruch 21,
**dadurch gekennzeichnet, dass**
ein Zufuhrgefäß (19) für das Medium (25) in der Medienleitung (15) vorhanden ist.

23. Vorrichtung (10) gemäß Anspruch 22,
**dadurch gekennzeichnet, dass**
ein erstes Ventil (18a) in der Medienleitung (15) zwischen dem Zufuhrgefäß (19) und der Kolben-Zylinder-Kombination (31 - 32) aufgenommen ist.

24. Vorrichtung (10) gemäß einem der Ansprüche 21 bis 23,
**dadurch gekennzeichnet, dass**
ein zweites Ventil (18b) in der Medienleitung (15) zwischen der Kolben-Zylinder-Kombination (31- 32) und dem aufblasbaren Ballonsystem (12) aufgenommen ist.

25. Vorrichtung (10) gemäß Anspruch 24,
**dadurch gekennzeichnet, dass**
sowohl das erste (18a) als auch das zweite (18b) Ventil durch die Beobachtungseinrichtung (16) aktiviert werden kann.

26. Vorrichtung (10) gemäß einem der vorangehenden Ansprüche,
**dadurch gekennzeichnet, dass**
die Strahlungsfreisetzungseinrichtung (20) als ein Nachführgerät ausgebildet ist.

27. Vorrichtung (10) gemäß einem der vorangehenden Ansprüche,
**dadurch gekennzeichnet, dass**
das druckbeaufschlagte Medium (25) ein Fluid, ein gasförmiges Medium oder eine Flüssigkeit ist, das/die radioaktive Partikel enthält.

## Revendications

1. Dispositif (10) pour le traitement par rayonnement d'un tissu prolifératif entourant une cavité (2) dans un corps d'animal (1) comprenant :
au moins un système de ballon gonflable (12) ayant une paroi de ballon pour le placement dans ladite cavité ;
des moyens de gonflage (30) pour gonfler et dégonfler ledit système de ballon (12) avec un milieu pressurisé (25) ;
des moyens d'apport de rayonnement (20) pour placer au moins une source émettrice d'énergie (22) dans ladite cavité (2) pour réaliser ledit traitement par rayonnement, ainsi que
des moyens de surveillance (16) pour surveiller l'état de gonflement du système de ballon gonflable (12),
**caractérisé en ce que** lesdits moyens de surveillance (16) sont agencés pour comparer l'état de gonflage effectif qui est surveillé avec un état de gonflage souhaité prédéterminé du système de ballon gonflable (12) et pour actionner le dispositif (10) en se basant sur les signaux de commande générés par suite de ladite comparaison de l'état de gonflage.

2. Dispositif (10) selon la revendication 1, **caractérisé en ce que** lesdits moyens d'apport de rayonnement (20) sont agencés pour rétracter ladite au moins une source émettrice d'énergie (22) de ladite cavité (2) en se basant sur des signaux de commande générés par lesdits moyens de surveillance (16) par suite de ladite comparaison de l'état de gonflage.

3. Dispositif (10) selon la revendication 1, **caractérisé en ce que,** ladite au moins une source émettrice d'énergie (22) est une source émettrice d'énergie activable et dans lequel lesdits moyens d'apport de rayonnement (20) sont agencés pour désactiver ladite au moins une source émettrice d'énergie dans ladite cavité (2) en se basant sur des signaux de commande générés par lesdits moyens de surveillance (16) par suite de ladite comparaison de l'état de gonflage.

4. Dispositif (10) selon la revendication 1, **caractérisé en ce que** lesdits moyens de gonflage (30) sont agencés pour gonfler et/ou dégonfler le système de ballon (12) en se basant sur des signaux de commande générés par lesdits moyens de surveillance (16) par suite de ladite comparaison de l'état de gonflage.

5. Dispositif (10) selon l'une quelconque des revendications 1 à 4, **caractérisé en ce que** lesdits moyens de surveillance (16) comprennent au moins un capteur de pression (17-17') pour générer des données de pression correspondant à la pression effective dudit milieu pressurisé (25) dans ledit système de ballon gonflé (12), lesdites données de pression étant utilisées pour ladite comparaison de l'état de gonflage.

6. Dispositif (10) selon la revendication 5, **caractérisé en ce que** les moyens de surveillance (16) sont agencés pour comparer lesdites données de pression à une largeur de bande de pression prédéterminée et pour actionner le dispositif (10) en se basant sur des signaux de commande générés par suite de ladite comparaison de pression.

7. Dispositif (10) selon la revendication 5 ou 6, **caractérisé en ce que** ledit au moins un capteur de pression (17') est positionné à l'intérieur du système de ballon (12).

8. Dispositif selon la revendication 5 ou 6, **caractérisé en ce que** ledit au moins un capteur de pression (17) est positionné à l'extérieur du système de ballon.

9. Dispositif (10) selon l'une quelconque des revendications 1 à 4, **caractérisé en ce que** lesdits moyens de surveillance (16) comprennent un dispositif d'imagerie (17") pour générer des données d'image correspondant au contour de paroi de ballon effectif du système de ballon gonflé (12), lesdites données d'image étant utilisées pour ladite comparaison de l'état de gonflage.

10. Dispositif (10) selon la revendication 9, **caractérisé en ce que** les moyens de surveillance (16) sont agencés pour comparer lesdites données d'image à un contour de paroi de ballon prédéterminé et pour actionner le dispositif en se basant sur les signaux de commande générés par suite de ladite comparaison de contour.

11. Dispositif (10) selon la revendication 9 ou 10, **caractérisé en ce que** les moyens de surveillance (16) sont agencés pour convertir lesdites données d'image obtenues avec ledit dispositif d'imagerie (17") en une image tridimensionnelle du contour de paroi de ballon effectif du système de ballon gonflé (12).

12. Dispositif (10) selon l'une quelconque des revendications 9 à 11, **caractérisé en ce que** ledit dispositif d'imagerie (17") est construit comme une sonde d'imagerie à ultrasons.

13. Dispositif (10) selon l'une quelconque des revendications 9 à 11, **caractérisé en ce que** ledit dispositif d'imagerie (17") est construit comme une caméra vidéo.

14. Dispositif (10) selon l'une quelconque des revendications 9 à 13, **caractérisé en ce que** ledit dispositif d'imagerie (17") est insérable à l'intérieur du système de ballon (12).

15. Dispositif (10) selon l'une quelconque des revendications 1 à 4, **caractérisé en ce que** lesdits moyens de surveillance (16) comprennent au moins un capteur de dose de rayonnement (40) pour générer des données de rayonnement correspondant à un rayonnement mesuré émis par ladite au moins une source émettrice d'énergie (22) qui est placée à l'intérieur de ladite cavité (2) et correspondant à la distance effective entre ledit au moins un capteur de dose de rayonnement (40) et ladite au moins une source émettrice d'énergie (22) à l'intérieur de ladite cavité (2), lesdites données de rayonnement étant utilisées pour ladite comparaison de l'état de gonflage.

16. Dispositif (10) selon la revendication 15, **caractérisé en ce que** les moyens de surveillance (16) sont agencés pour comparer lesdites données de rayonnement avec une distance souhaitée prédéterminée entre ledit au moins un capteur de dose de rayonnement (40) et ladite au moins une source émettrice d'énergie (22) à l'intérieur de ladite cavité (2) et pour actionner le dispositif (10) en se basant sur des signaux de commande générés par suite de ladite comparaison de rayonnement.

17. Dispositif (10) selon la revendication 15 ou 16, **caractérisé en ce que** ledit au moins un capteur de rayonnement (40) est raccordé à la paroi interne du système de ballon (12).

18. Dispositif (10) selon la revendication 15 ou 16, **caractérisé en ce que** ledit au moins un capteur de rayonnement (40) est raccordé à la paroi externe du système de ballon (12).

19. Dispositif (10) selon l'une quelconque des revendications précédentes, **caractérisé en ce que** les moyens de gonflage (30) comprennent une combinaison piston-cylindre (31-32) ayant un cylindre (31) et un piston (32) mobile logé dans ledit cylindre.

20. Dispositif (10) selon la revendication 19, **caractérisé en ce que** lesdits moyens de gonflage (30) comprennent des moyens d'entraînement de piston (35) pour déplacer ledit piston (32) à l'intérieur dudit cylindre (31) en se basant sur des signaux de commande générés par lesdits moyens de surveillance (16).

21. Dispositif (10) selon la revendication 20, **caractérisé en ce qu'**une conduite de milieu (14-15) reliant le cylindre (31) au ballon gonflable (12) est présente.

22. Dispositif (10) selon la revendication 21, **caractérisé en ce qu'**une cuve d'alimentation (19) pour ledit milieu (25) est présente dans ladite conduite de milieu (15).

23. Dispositif (10) selon la revendication 22, **caractérisé en ce qu'**une première soupape (18a) est logée dans ladite conduite de milieu (15) entre ladite cuve d'alimentation (19) et ladite combinaison piston-cylindre (31-32).

24. Dispositif (10) selon l'une quelconque des revendications 21 à 23, **caractérisé en ce qu'**une seconde soupape (18b) est logée dans ladite conduite de milieu (15) entre ladite combinaison piston-cylindre (31-32) et ledit système de ballon gonflable (12).

25. Dispositif (10) selon la revendication 24, **caractérisé en ce qu'**à la fois ladite première (18a) et ladite seconde (18b) soupape peuvent être actionnées par lesdits moyens de surveillance (16).

26. Dispositif (10) selon l'une quelconque des revendications précédentes, **caractérisé en ce que** lesdits moyens d'apport de rayonnement (20) sont construits comme un appareil post-charge.

27. Dispositif (10) selon l'une quelconque des revendications précédentes, **caractérisé en ce que** ledit milieu pressurisé (25) est un fluide, un milieu gazeux ou un liquide contenant des particules radioactives.
